# EUROPÄISCHE PATENTSCHRIFT

(11) **EP 1 987 129 B1**
(45) Veröffentlichungstag und Bekanntmachung des Hinweises auf die Patenterteilung: **02.04.2014**
(21) Anmeldenummer: 06829215.0
(22) Anmeldetag: 30.11.2006
(51) Int. Cl.: C12M 1/113

(54) **FERMENTATIONSEINRICHTUNG MIT GEKOPPELTEM SUBSTRAT- UND SEDIMENTTRANSPORT UND VERFAHREN ZUM BETRIEB DER FERMENTATIONSEINRICHTUNG**
FERMENTATION DEVICE COMPRISING A COUPLED SUBSTRATE AND SEDIMENT TRANSPORT MECHANISM AND METHOD FOR OPERATING THE FERMENTATION DEVICE
DISPOSITIF DE FERMENTATION A TRANSPORT COUPLE DU SUBSTRAT ET DES SEDIMENTS ET PROCEDE PERMETTANT DE FAIRE FONCTIONNER LEDIT DISPOSITIF

(30) Priorität: 05.12.2005 DE 102005057978
(43) Veröffentlichungstag der Anmeldung: 05.11.2008
(73) Patentinhaber: STRABAG Umweltanlagen GmbH, 01069 Dresden (DE)
(72) Erfinder: BÜCHNER, Thomas, 01731 Kreischa (DE); LANGHANS, Gerhard, 01159 Dresden (DE); HALLER, Urs, CH-332 Urtenen-Schönbühl (CH); SICKINGER, Roland, 72147 Nehren (DE)
(86) Internationale Anmeldenummer: PCT/EP2006/011518
(87) Internationale Veröffentlichungsnummer: WO 2007/065597

(56) Entgegenhaltungen:
- EP-A2- 0 617 120
- DE-A1- 3 239 304
- JP-A- 10 192 677

## Beschreibung

Die Erfindung betrifft eine Fermentationseinrichtung zum biologischen Abbau von organisches Material enthaltendem Substrat und zur Gewinnung des beim Abbau entstehenden Biogases mit einem längserstreckten geschlossenen Behälter mit einer stirnseitigen Einbringöffnung für das Substrat und einer Austragsöffnung für behandeltes Substrat am gegenüberliegenden Ende des Behälters sowie mindestens einer Abzugsöffnung für Biogas, wobei der Behälter mehrere Reaktionsvolumenzellen aufweist, die jeweils mehrere individuell angetriebene Rühreinrichtungen mit kreisförmig bewegten Rührflügeln und quer zur Längserstreckung des Behälters liegenden Rührerwellen zum Durchmischen des Substrats enthalten, sowie ein Verfahren zum Betrieb der Fermentationseinrichtung.

Bei der Fermentation von organisches Material enthaltendem Substrat, z.B. von Bioabfall, Rückständen aus der Nahrungsmittelproduktion oder nachwachsenden Rohstoffen, unterscheidet man zwischen der sogenannten Nassvergärung und der sogenannten Trockenvergärung. Während bei der Nassvergärung eine fließfähige Suspension aus dem Substrat hergestellt wird, die in einem Biogasreaktor, z.B. einem Schlaufenreaktor mit innenliegender Schlaufenströmung, behandelt wird, wird das Substrat bei der Trockenvergärung z.B. an einem Ende in einen geschlossenen liegenden Behälter eingebracht, mittels einer Rühreinrichtung unter Biogasbildung umgewälzt und das behandelte Substrat am anderen Ende aus dem Behälter abgezogen.

Die Verfahren der Trockenvergärung werden alternativ zu denen der Nassvergärung eingesetzt, wenn das anaerob zu behandelnde Substrat sehr-feststoffreich ist und deshalb die notwendige Verdünnung bis in den Betriebsbereich der Nassverfahren mit ihren Feststoffkonzentrationen von weniger als ca. 15% unwirtschaftlich ist, bzw. wenn die häufig notwendige, aber kostenaufwendige Störstoffentfrachtung bei Nassverfahren aus sonstigen prozesstechnischen Gründen nicht erforderlich ist. Trockenvergärungen werden bevorzugt bei Feststoffgehalten von gleich oder kleiner als 35% im Fermentereintritt gefahren und können als stehende oder liegende Reaktoren ausgeführt sein.

Ein Vorteil der Trockenvergärung beim Einsatz in der Abfall- und Restmüllbehandlung ist der vergleichsweise geringe Aufbereitungs- und Konditionierungsaufwand für die angenommenen Eingangsstoffe vor der Vergärung. Nach einer Abreicherung grober Störstoffe und anschließender Zerkleinerung der Fraktion für die Vergärung auf 20 bis 60 mm ist in der Regel vor der Dosierung in den Gärreaktor keine weitere Störstoffentfrachtung für z.B. Holz, Plastik, Glas sowie Sand und Steine notwendig.

Erkauft wird dieser geringe technologische Aufwand für die Einsatzvorbehandlung mit möglichen Problemen im Fermenter selbst. Durch den biochemischen Umsatz organischer Verbindungen im Gärmedium über die Fermenterlänge verringern sich zwischen Einbringöffnung und Austragsöffnung die Feststoffkonzentrationen von etwa 35% Trockensubstand auf kleiner als 20% Trockensubstanz und die Viskosität von 30.000 bis 60.000 mPas auf bis zu kleiner 5.000 mPas. Waren in dem feststoffreichen Einsatz die weiter oben genannten sedimentierfähigen und flotierfähigen Störstoffe weitgehend lagestabil verteilt, werden sie durch die sich verändernden Stoffeigenschaften des Gärmediums im Fermenter mobilisiert, was tendenziell zur Ausbildung von Sink- und Schwimmschichten führt. Die üblicherweise in Fermentern angeordneten Rührorgane zur Verbesserung von Stoffübergang und Medienentgasung unterstützen diese fraktionierte Entmischung noch.

Deshalb finden sich insbesondere in liegenden Trockenfermentern neben Rührorganen noch besondere Vorrichtungen hauptsächlich zum Transport und zur Entnahme von Sedimenten.

So sind in der EP 0 617 120 B1 spezielle Scharren beschrieben, die am Fermenterboden durch über Schubstangen übertragene Bewegungen die Sedimente zum Reaktorende in Richtung dünner werdendes Medium transportieren.

Bei dieser bekannten Konstruktion werden also für die Durchmischung und die Störstoffentnahme getrennte Ausrüstungen benötigt, was den technologischen und betriebstechnischen Aufwand erhöht. Insbesondere Wartung und Störungsbehebung bringen erhebliche Probleme, da sie mit einem Abfahren des Fermenters verbunden sein können.

In der DE-A-3239304 wird ein Naßfermenter beschrieben, in dem in einem liegenden zylindrischen Reaktor Biogas aus dünnflüssiger Gülle erzeugt wird. Der Naßfermenter weist senkrecht zur Erstreckung des Behälters waagerecht liegende, gegensinnig arbeitende Rührwerke zum Rühren der Gülle auf.

Der vorliegenden Erfindung liegt die Aufgabe zugrunde, eine Fermentationseinrichtung der eingangs genannten Art sowie ein Verfahren zum Betrieb der Fermentationseinrichtung so auszugestalten, dass neben der eigentlichen Homogenisier- und Entgasungsfunktion der Rührwerke ohne Beeinträchtigung der Pfropfenstromcharakteristik für den Feststoffstrom zwischen Fermenterein- und austritt gleichzeitig eine Sedimentbewegung zum Fermenterende induziert und die Ausbildung verfestigter Schwimmdecken unterdrückt wird.

Diese Aufgabe wird erfindungsgemäß bei der Fermentationseinrichtung dadurch gelöst, dass die Rührerwellen mit Achsabständen angeordnet sind, die kleiner sind als der Rührdurchmesser, so dass die Rührflügel beim Drehen der Rührerwellen einen Überlappungsbereich überstreichen.

Die Erfindung geht dabei von dem Stand der Technik aus, wie er in der EP 0 617 120 B1 beschrieben ist. Diese bekannte Fermentationseinrichtung weist separat ansteuerbare, querliegende Rührwerkseinheiten auf, die den Fermenter in eine Reihe von hintereinander geschalteten fiktiven Reaktionsvolumenzellen (Rührzellen) zerlegen. Die Schaufelrad-ähnlichen Balkenrührwerke bewirken in ihrer jeweiligen Rührzelle einen vertikalen Medienaustausch einschließlich einer Schwimmdeckenzerteilung durch die aus der Flüssigkeit auftauchenden Rührbalken, während der horizontale Pfropfenstrom durch die Kombination von Gärgutentnahme und Substratdosierung erzeugt wird. Der Sedimenttransport erfolgt dabei separat mittels eines Schubrahmens. Die Rührerwellen sind bei der bekannten Fermentationseinrichtung im Fermenter so angeordnet, dass der Abstand der Rührerwellen zueinander größer ist als der Rührerdurchmesser.

Aus theoretischen Überlegungen, die experimentell bestätigt wurden, hat sich überraschend gezeigt, dass dieses Rührsystem auch für den gezielten Sedimenttransport längs des Fermenterbodens genutzt werden kann. Dazu wird der von Wanderdünen bekannte Effekt der horizontalen Verschiebung durch Materialtransport von der Luv- auf die Leeseite ausgenutzt. Transportvehikel sind dabei die Rührflügel, die bei entsprechend ausgelegtem Abstand zum Reaktorboden die Sedimente zu einer Düne quer zur Längsachse des Behälters zusammenschieben. Bei den derzeitig üblichen Rührergeometrien und Achsabständen verläuft dieser Transportvorgang jedoch ineffektiv und kann völlig zum Stillstand kommen bzw. sogar zum Festfahren der Rührwerke führen in Abhängigkeit von Trockensubstanzgehalt, Medienviskosität und Störstoffanteil.

In einer besonders bevorzugten Ausführungsform der Erfindung weisen die Rührflügel an ihren äußeren Enden Rührbalken auf, die über die Breite des Behälters geteilt und wechselseitig versetzt angeordnet sind. Wie sich nämlich gezeigt hat, sollten die Sedimentdünen klein genug bleiben, um beweglich zu sein. Dazu werden die Rührbalken über die Reaktorbreite geteilt und wechselseitig bevorzugt in Winkeln unter 120 Grad oder 90 Grad versetzt auf der Rührerwelle angeordnet. In Kombination mit der speziellen Geometrie des Rührsystems, bei der die Rührerwellen mit Achsabständen angeordnet sind, die kleiner sind als der Rührerdurchmesser, lässt sich die maximale Höhe der Sedimentaufwerfung in den Toträumen zwischen den rotierenden Rührbalken geometrisch in der gewünschten Weise vorgeben. Bei gleichsinniger Drehrichtung der benachbarten Rührwerke wird Sediment auf der einen Seite angehäuft und von dem versetzt laufenden, kämmenden Rührbalken des Folgerührwerks auf der anderen Seite wieder abgetragen und über den Boden bis zur nächsten Düne transportiert. Durch die in Querrichtung geteilten und auf der Welle versetzt angeordneten Rührbalken wird vermieden, dass der Rührbalken über die ganze Breite gleichzeitig in das Sediment eintaucht und es dadurch zu Schäden infolge mechanischer Überlastung kommt. Außerdem können sich seitlich keine Grobstoffe zwischen Rührbalken und Behälterwand verklemmen.

Zweckmäßigerweise ist im Austragsbereich der Fermentationseinrichtung ein bodennaher Saugstutzen zum Abzug der dorthin transportierten Sedimente angeordnet. Insbesondere für die Behandlung von Gärmedien mit speziellen Sedimenteigenschaften ist gemäß einer Weiterbildung des Erfindungsgedankens bauseitig im Austragsbereich der Fermentationseinrichtung eine Sammeltasche mit geneigten Wänden ausgebildet, an deren tiefsten Punkt der Saugstutzen endet.

Die Erfindung betrifft ferner ein Verfahren zum biologischen Abbau von organisches Material enthaltendem Substrat und zur Gewinnung des beim Abbau entstehenden Biogases, wobei das Substrat in einen längserstreckten geschlossenen Behälter eingebracht wird, im Behälter mehrere Reaktionszonen durchströmt, in denen es mittels jeweils individuell angetriebener Rühreinrichtungen mit kreisförmig um quer zur Längserstreckung des Behälters liegende Achsen bewegte Rührflügeln umgewälzt wird und Abbauprodukte aus dem Behälter abgezogen werden.

Verfahrensseitig wird die gestellte Aufgabe dadurch gelöst, die Rührflügel benachbarter Rührwerke, die mit Achsabständen angeordnet sind, die kleiner sind als der Rührdurchmesser, so gesteuert werden, dass die Rührflügel auf Lücke arbeiten. Dadurch wird sichergestellt, dass sich die Rührflügel benachbarter Rührwerke nicht gegenseitig blockieren.

Die Rührflügel benachbarter Rührwerke werden gleichsinnig bewegt. Durch die gleichsinnige Drehrichtung wird Sediment auf der einen Seite angehäuft und von dem versetzt laufenden, kämmenden Rührbalken des benachbarten Rührwerks auf der anderen Seite wieder abgetragen und über den Boden bis zur nächsten Düne transportiert.

Die Erfindung eignet sich für alle Fermentationseinrichtungen, die einen liegenden Behälter mit querliegenden Rühreinrichtungen aufweisen: Insbesondere bei der Behandlung von Substrat, das eine hohe Sedimentierneigung besitzt, kann die Erfindung mit großem Vorteil angewandt werden.

Insgesamt wird mit der Erfindung mit geringem Investitionsaufwand eine technisch elegante Lösung zur gleichzeitigen Durchmischung von Fermentationsgut und Austrag von abgelagerten Sedimenten im Fermenter zur Verfügung gestellt. Ein besonderer Vorteil besteht dabei darin, dass die Pfropfenstromcharakteristik bezüglich des zu behandelnden Substrats in der Fermentationseinrichtung durch den Sedimentaustrag nicht gestört wird.

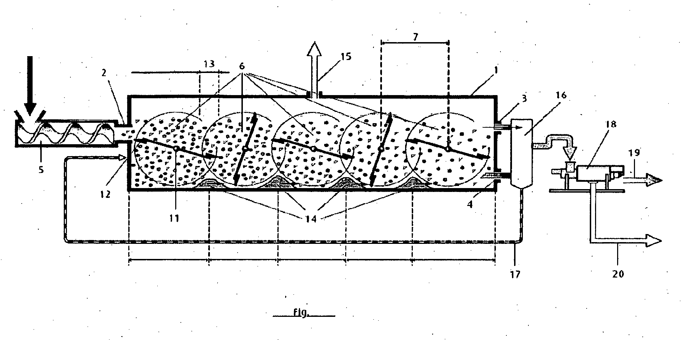

Im Folgenden soll die Erfindung anhand eines in der Figur schematisch dargestellten Ausführungsbeispiels näher erläutert werden:.

Die Figur zeigt eine Fermentationseinrichtung mit einem liegenden geschlossenen Behälter 1, der am stirnseitigen Ende eine Eintragsöffnung 2 für zu behandelndes Substrat und am gegenüberliegenden Ende Austragsöffnungen 3 und 4 aufweist. Das Substrat, das beispielsweise aus Bioabfall oder nachwachsenden Rohstoffen besteht, wird über eine Zuführschnecke 5 in die Eintragsöffnung 2 des Behälters 1 eingebracht. Im Behälter 1 sind quer zur Behälterachse angeordnete Rühreinrichtungen 6 angeordnet, die jeweils eine quer zur Behälterachse ausgerichtete Rührerwelle 11 aufweisen, welche individuell angetrieben wird. An den Rührflügeln der Rührwerke 6 sind Rührbalken 12 angebracht, die über die Behälterbreite geteilt und wechselseitig in Winkeln unter 120 Grad versetzt auf der Rührerwelle angeordnet sind. Außerdem sind die Rührerwellen 11 mit Achsabständen angeordnet, die kleiner sind als der Rührerdurchmesser, so dass es zu einem Kämmen der Rührbalken 12 kommt. Der Überlappungsbereich (Kämmzone) benachbarter Rührwerke 6 ist mit der Bezugsziffer 13 bezeichnet. Durch geometrische Festlegung der Achsabstände 6 sowie der Rührerdurchmesser in der Konstruktionsphase lässt sich die maximale Höhe der Sedimentaufwerfung 14 in den Toträumen zwischen den rotierenden Rührbalken 12 technologisch in der gewünschten Weise vorgeben. Bei gleichsinniger Drehrichtung der benachbarten Rührwerke 6 wird Sediment auf der einen Seite angehäuft und von dem versetzt laufenden, kämmenden Rührbalken des Folgerührwerks auf der anderen Seite wieder abgetragen und über den Boden bis zur nächsten Düne transportiert. Das während der Behandlung im Behälter erzeugte Biogas wird über eine Abgasöffnung 15 aus dem Behälter 1 abgezogen. Die zum Fermenterende transportierten Sedimentdünen 14 gelangen in den Bereich der Austragsöffnung 4, wo Trockensubstanz und sedimentangereicherte Bodenfraktion aus dem Behälter 1 abgesaugt werden. Feststoffarme Flüssigkeit wird im oberen Bereich des Behälters über die Austragsöffnung 3 abgeführt. In einem nachgeschaltetem Behälter 16 wird Gärgut abgetrennt und einer Entwässerungspresse 18 zugeführt. Abgeschiedene Gärreststoffe werden schließlich über eine Ableitung 19 abgezogen, während Presswasser z.B. als Flüssigdünger über eine Leitung 20 abgeführt wird. Über eine Rücklaufleitung 17 wird Faulgut und Presswasser zum stirnseitigen Ende des Behälters 1 bedarfsweise zurückgeführt.

## Patentansprüche

1. Trockenfermentationseinrichtung zum biologischen Abbau von organisches Material enthaltendem Substrat und zur Gewinnung des beim Abbau entstehenden Biogases mit einem längserstreckten geschlossenen Behälter (1) mit einer stirnseitigen Einbringöffnung (2) für das Substrat und einer Austragsöffung (3, 4) für behandeltes Substrat am gegenüberliegenden Ende des Behälters (1) sowie mindestens einer Abzugsöffnung (15) für Biogas, wobei der Behälter (1) mehrere Reaktionsvolumenzellen aufweist, die jeweils mehrere individuell angetriebene Rühreinrichtungen (6) mit kreisförmig gleichsinnig bewegten Rührflügeln (7) und quer zur Längserstreckung des Behälters (1) liegenden Rührerwellen (11) zum Durchmischen des Substrats enthalten, **dadurch gekennzeichnet, dass** die Rührerwellen (11) mit Achsabständen (7) angeordnet sind, die kleiner sind als der Rührerdurchmesser, so dass die Rührflügel beim Drehen der Rührerwellen (11) einen Überlappungsbereich (13) überstreichen.

2. Fermentationseinrichtung nach Anspruch 1, **dadurch gekennzeichnet, dass** die Rührflügel an ihren äußeren Enden Rührbalken (12) aufweisen, die über die Breite des Behälters (1) geteilt und wechselseitig versetzt angeordnet sind.

3. Fermentationseinrichtung nach Anspruch 1 oder 2, **dadurch gekennzeichnet, dass** im Austragsbereich der Fermentationseinrichtung ein bodennaher Saugstutzen (4) zum Abzug von Sedimenten angeordnet ist.

4. Fermentationseinrichtung nach Anspruch 3, **dadurch gekennzeichnet, dass** der Behälter (1) im Austragsbereich der Fermentationseinrichtung eine Sammeltasche mit geneigten Wänden aufweist, an deren tiefsten Punkt der Saugstutzen (4) endet.

5. Trockenfermentationsverfahren zum biologischen Abbau von organisches Material enthaltendem Substrat und zur Gewinnung des beim Abbau entstehenden Biogases, wobei das Substrat in einen längserstreckten geschlossenen Behälter (1) eingebracht wird, im Behälter (1) mehrere Reaktionszonen durchströmt, in denen es mittels jeweils individuell angetriebener Rühreinrichtungen (6) mit kreisförmig um quer zur Längserstreckung des Behälters (1) liegende Achsen (11) gleichsinnig bewegte Rührflügeln umgewälzt wird und Abbauprodukte aus dem Behälter (1) abgezogen werden, **dadurch gekennzeichnet, dass** die Rührflügel benachbarter Rührwerke (6), die mit Achsabständen angeordnet sind, die kleiner sind als der Rührerdurchmesser, so gesteuert werden , dass die Rührflügel auf Lücke arbeiten.

## Claims

1. Dry fermentation device for the biodegradation of substrate containing organic material and for recovering the biogas produced during degradation, with an elongate, closed container (1) having an end-side introduction opening (2) for the substrate and a discharge opening (3, 4) for treated substrate at the opposite end of the container (1), and at least one take-off opening (15) for biogas, wherein the container (1) has a plurality of reaction volume cells which each contain a plurality of individually driven stirring devices (6) having stirring blades, which move in a circular manner in the same direction, and stirrer shafts (11), which lie transversely with respect to the longitudinal extent of the container (1), for thoroughly mixing the substrate, **characterized in that** the stirrer shafts (11) are arranged at axial distances (7) which are smaller than the stirrer diameter, and therefore the stirring blades move through an overlapping region (13) during the rotation of the stirrer shafts (11).

2. Fermentation device according to Claim 1, **characterized in that** the outer ends of the stirring blades have stirring bars (12) which are divided over the width of the container (1) and are arranged offset in an alternating manner.

3. Fermentation device according to Claim 1 or 2, **characterized in that** a suction connecting pipe (4) in the vicinity of the base is arranged in the discharge region of the fermentation device in order to take off sediment.

4. Fermentation device according to Claim 3, **characterized in that**, in the discharge region of the fermentation device, the container (1) has a collecting pocket with inclined walls, with the suction connecting pipe (4) ending at the lowermost point of said collecting pocket.

5. Dry fermentation method for the biodegradation of substrate containing organic material and for recovering the biogas produced during degradation, wherein the substrate is introduced into an elongate, closed container (1), flows through a plurality of reaction zones in the container (1), in which reaction zones said substrate is circulated by means of stirring devices (6) which are each driven individually and have stirring blades moving circularly in the same direction about axes (11) lying transversely with respect to the longitudinal extent of the container (1), and degradation products are taken off out of the container (1), **characterized in that** the stirring blades of adjacent stirring mechanisms (6), which are arranged at axial distances which are smaller than the stirrer diameter, are controlled in such a manner that the stirring blades operate in a staggered manner.

## Revendications

1. Dispositif de fermentation à sec en vue de la décomposition biologique d'un substrat contenant de la matière organique et de la récupération du biogaz produit lors de la décomposition, le dispositif présentant
un récipient (1) fermé et allongé doté d'une ouverture frontale (2) d'amenée de substrat et, à l'extrémité opposée du récipient (1), d'une ouverture de sortie (3, 4) pour le substrat traité, ainsi qu'au moins une ouverture (15) d'extraction de biogaz,
le récipient (1) présentant plusieurs volumes cellulaires de réaction qui contiennent chacun plusieurs dispositifs de brassage (6) entraînés séparément et dotés d'ailes de brassages déplacées en cercle dans le même sens et d'arbres de brassage (11) disposés transversalement par rapport à l'extension longitudinale du récipient (1) pour brasser intimement le substrat,
**caractérisé en ce que**
les arbres de brassage (11) sont disposés à des écarts axiaux (7) inférieurs au diamètre du dispositif de brassage de telle sorte que les ailes de brassage balayent une zone de superposition (13) lors de la rotation des arbres de brassage (11).

2. Dispositif de fermentation selon la revendication 1, **caractérisé en ce que** les ailes de brassage présentent à leur extrémité extérieure des barres de brassage (12) qui sont réparties sur la largeur du récipient (1) et décalées en alternance.

3. Dispositif de fermentation selon les revendications 1 ou 2, **caractérisé en ce qu'**une tubulure d'aspiration (4) proche du sol est prévue pour l'extraction des sédiments dans la zone d'extraction du dispositif de fermentation.

4. Dispositif de fermentation selon la revendication 3, **caractérisé en ce que** dans la zone d'extraction du dispositif de fermentation, le récipient (1) présente une poche de collecte dotée de parois inclinées qui se terminent en leur point le plus bas par la tubulure d'aspiration (4).

5. Procédé de fermentation à sec en vue de la décomposition biologique de substrats contenant de la matière organique et de récupération du biogaz produit lors de la décomposition, le substrat étant amené dans un récipient (1) fermé et allongé, traversant dans le récipient (1) plusieurs zones de réaction dans lesquelles il est mis en circulation au moyen d'un dispositif de brassage (6) doté d'ailes de brassage déplacées en cercle autour d'axes (11) disposés transversalement par rapport à l'extension longitudinale du récipient (1),
les produits de décomposition étant extraits du récipient (1),
**caractérisé en ce que**
les ailes de brassage de dispositifs (6) de brassage voisins, disposées à écart axial inférieur au diamètre de brassage, sont commandées de telle sorte que les ailes de brassage travaillent sur les interstices.
